# EUROPEAN PATENT APPLICATION

(11) **EP 2 599 499 A1**
(43) Date of publication of application: **05.06.2013**
(21) Application number: 13155903.1
(22) Date of filing: 04.03.2009
(51) Int. Cl.: A61K 45/06, A61P 35/00, A61K 31/5377

(54) **Use of pyrimidine derivatives for the treatment of EGFR dependent diseases or diseases that have acquired resistance to agents that target EGFR family members**

(30) Priority: 05.03.2008 EP 08152326
(62) Divisional of application: 09716778.7
(71) Applicant: Novartis AG, 4056 Basel (CH)
(72) Inventor: García-Echeverría, Carlos, 92210 Saint-Cloud (FR); Maira, Sauveur-Michel, 68440 Habsheim (FR)
(74) Representative: Rudge, Sewkian

(57) **Abstract**

The present invention relates to the use of compounds of formula (I) 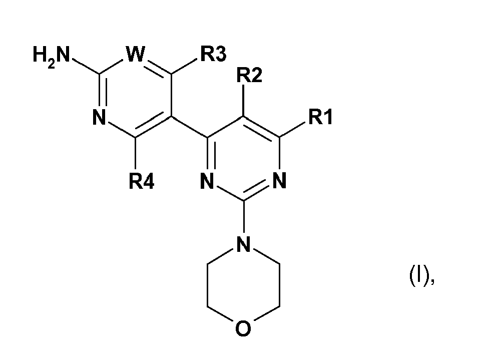 in the treatment of Epidermal Growth Factor Receptor (EGFR) family members dependent diseases or diseases that have acquired resistance to agents that target EGFR family members, use of said compounds for the manufacture of pharmaceutical compositions for the treatment of said diseases, combinations of said compounds with EGFR modulators for said use, methods of treating said diseases with said compounds and pharmaceutical preparations for the treatment of said diseases comprising said compounds alone or in combination, especially with an EGFR modulator.

## Description

The present invention relates to the use of specific pyrimidine derivatives in the treatment of Epidermal Growth Factor Receptor (EGFR) family members (including EGFR1 also known as HER1 or Erb-B1; EGFR2 also known as HER2 or Erb-B2; and EGFR3 also known as HER3 or Erb-B3) dependent diseases or diseases that have acquired resistance to agents that target EGFR family members, use of said compounds for the manufacture of pharmaceutical compositions for the treatment of said diseases, combinations of said compounds with EGFR modulators for said use, methods of treating said diseases with said compounds, and pharmaceutical preparations for the treatment of said diseases comprising said compounds, alone or in combination, especially with an EGFR modulator.

Somatic mutations in the tyrosine kinase domain of EGFR has been associated with the clinical response to EGFR tyrosine kinase inhibitor such as Gefitinib (Iressa®) or Erlotinib (Tarceva®) (Paez et al., EGFR mutations in lung cancer: correlation with clinical response to gefitinib therapy, science, vol 304, 1497-1500)*.* Acquired resistance to EGFR modulators occurs in patients who initially responded clinically to therapy, but then developed progressive tumors. Refractory response to EGFR kinase inhibitors is exemplified with the secondary resistant mutation T790M (Kobayashi et al.; EGFR mutation and resistance of non-small cell lung cancer to gefitinib, N. Engl J Med, Vol 352, 786-792), which is comparable to the resistance mutation(s) observed for Gleevec/Glivec or Dasatinib in chronic myelogenous leukemia (CML) (Gorre et al.; Bcr-Abl point mutants isolated from patients with imatinib mesylate resistant chronic leukemia reamin sensitive to inhibitors of the Bcr-Abl chaperone heat shock protein 90, Blood, vol 100, 3041-3044) or GIST patients (Antonescu et al.; Acquired resistance to Imatinib in gastrointestinal stromal tumors occurs through secondary gene mutation, Clin Cancer Res, Vol 11, 4182-4190)*.*

Evidences that activation of the PI3K pathway downstream of activated EGFR exists in the literature. Thus, genetic ablation of the PI3K catalytic subunit (p110) in mouse embryo fibroblast renders cells resistant for transformation by an activated form of EGFR (Zhao et al.; The p110 alpha isoform of P13K is essential for proper growth factor signaling and oncogenic transformation, PNAS, vol 103, 16296-16300)*.* HER3 (ErbB-3), one of the four member of the EGFR family and partner of HER1 (EGFR1) is often overexpressed in EGFR inhibitors sensitive tumors, and that is correlated with constitutive PI3K recruitment and activation (Engelman et al.; ErbB-3 mediates phosphoinositide 3-kinase activity in gefitinib-sensitive non small cell lung cancer cell lines, PNAS vol 102, 3788-3793*; Sergina et al.; Escape from HER-family tyrosine kinase inhibitor therapy by the kinase-inactive HER 3*). The genetic and biochemical characterization of tumor biopsies and tumor cell lines harboring EGFR amplification and EGFR inhibitor resistance have revealed a constitutive activation status of the PI3K pathway (Engelman et al.; Allelic disruption obscures detection of a biologically significant resistance mutation in EGFR amplified lung cancer, The Journal of Clinical Investigation, vol 116, 2695-2706).

Surprisingly, it has been found that specific pyrimidine derivatives, which have been described in WO07/084786 provoke strong anti-proliferative activity and an in vivo antitumor response of breast and lung cancer cell lines with amplified EGFRs and/or mutated EGFR1 as single agent and in combination with EGFR kinase modulators. Therefore, said compounds are usefull for the treatment of EGFR dependent disease.

Specific pyrimidine derivatives which are suitable for the present invention, their preparation and suitable pharmaceutical formulations containing the same are described in WO07/084786 and include compounds of formula I 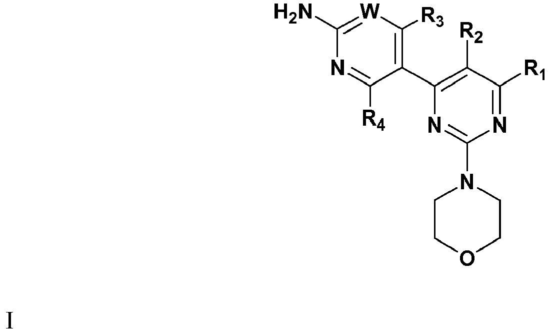 or a stereoisomer, tautomer, or pharmaceutically acceptable salt thereof, wherein, W is CR_{w} or N, wherein R_{w} is selected from the group consisting of
(1) hydrogen,
(2) cyano,
(3) halogen,
(4) methyl,
(5) trifluoromethyl,
(6) sulfonamido;
R₁ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₁ₐ,
(13) -CO₂R₁ₐ,
(14) -CONR₁ₐR_{1b},
(15) -NR₁ₐR_{1b},
(16) -NR₁ₐCOR_{1b},
(17) -NR₁ₐSO₂R_{1b},
(18) -OCOR₁ₐ,
(19) -OR₁ₐ,
(20) -SR₁ₐ,
(21) -SOR₁ₐ,
(22) -SO₂R₁ₐ, and
(23) -SO₂NR₁ₐR_{1b},
wherein R₁ₐ, and R_{1b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl;
R₂ is selected from the group consisting
(1) hydrogen,
(2) cyano,
(3) nitro,
(4) halogen,
(5) hydroxy,
(6) amino,
(7) substituted and unsubstituted alkyl,
(8) -COR₂ₐ, and
(9) -NR₂ₐCOR_{2b},
wherein R₂ₐ, and R_{2b} are independently selected from the group consisting of
(a) hydrogen, and
(b) substituted or unsubstituted alkyl;
R₃ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₃ₐ,
(13) -NR₃ₐR_{3b},
(14) -NR₃ₐCOR_{3b},
(15) -NR₃ₐSO₂R_{3b},
(16) -OR₃ₐ,
(17) -SR₃ₐ,
(18) -SOR₃ₐ,
(19) -SO₂R₃ₐ, and
(20) -SO₂NR₃ₐR_{3b},
wherein R₃ₐ, and R_{3b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl; and
R₄ is selected from the group consisting of
(1) hydrogen, and
(2) halogen.

The radicals and symbols as used in the definition of a compound of formula I have the meanings as disclosed in WO07/084786 which publication is hereby incorporated into the present application by reference.

A preferred compound of the present invention is a compound which is specifically described in WO07/084786.

A very preferred compound of the present invention is 5-(2,6-di-morpholin-4-yl-pyrimidin-4-yl)-4-trifluoromethyl-pyridin-2-ylamine (Compound A). The synthesis of 5-(2,6-di-morpholin-4-yl-pyrimidin-4-yl)-4-trifluoromethyl-pyridin-2-ylamine is described in WO07/084786 as Example 10.

Compounds that target members of the EGFR family according to the present invention include EGFR family kinase modulators, compounds that alter EGFR expression levels or elicit a cellular immune reponse linked to the expression of EGFR family members in the tumor cells. Preferrable EGFR modulators exhibit their activity as inhibitors of EGFR functional activity. Compounds that target members of the EGFR family according to the present invention include without limitation gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

### Short description of the figures

Figure 1 shows the antitumor activity of Compound A against the EGFR inhibitor resistant NSCLC cell line NCI-H1975.
   Female Harlan athymic mice (n = 6), bearing s.c NCI-H1975 tumors are treated p.o. with the PI3K inhibitor Compound A at 50mg/kg every day for the duration of the treatment. * p<0.05 (Dunnet's vs controls).
Figure 2 shows the mean body weight of vehicle and Compound A treated groups during the efficacy in vivo study performed with mice bearing the s.c. the EGFR inhibitor resistant NSCLC cell line NCI-H1975.

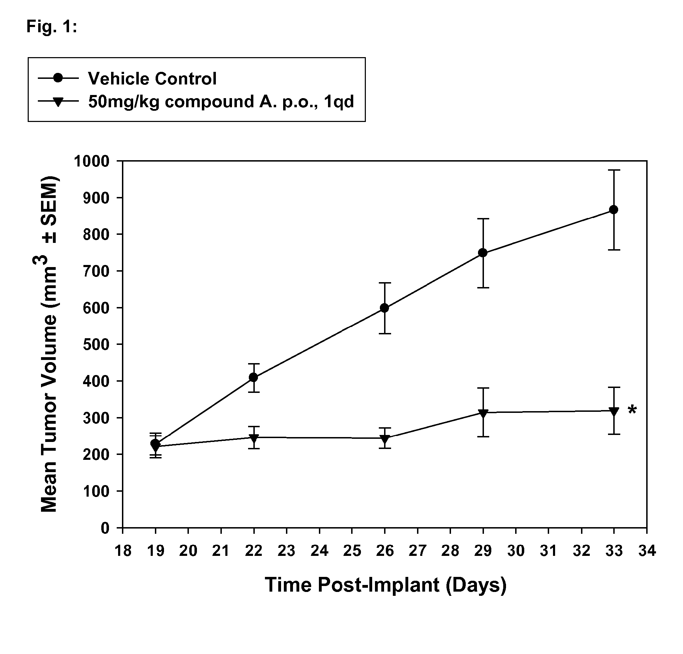

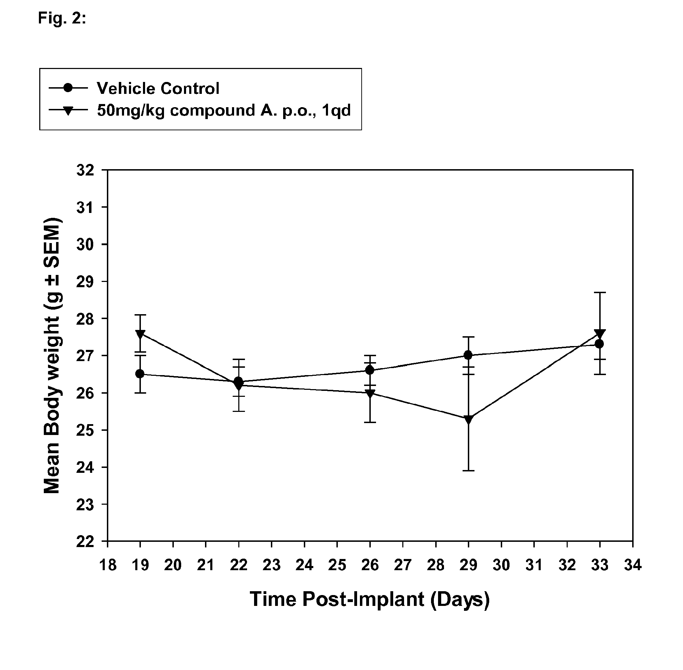

Non small cell lung carcinoma cell lines with EGFR mutant forms, but refractory to EGFR inhibition therapy, are valuable models to test the sensitivity of PI3K inhibitors like the compounds of formula I in such genetic background. The NCI-H1975 cell line is such a model as is highly tumorigenic in vivo. Moreover, it contains HER1 bearing the T790M gatekeeper mutation that renders the kinase resistant to catalytic inhibition by compounds such as gefitinib. The *in vivo* antitumor activity of PI3K inhibitors like the compounds of formula I is tested against this EGFR driven and EGFR tyrosine kinase inhibitor resistant tumor model (Figure 1). Surprisingly administration of Compound A causes *in vivo* tumor growth inhibition. Compound A is well tolerated - no statistically significant difference in body weight between the control and treatment groups can be observed (Figure 2).

A compound of formula I, especially Compound A, is therefore useful for the treatment of such EGFR dependent diseases, especially malignancies, or EGFR family members acquired resistance driven diseases. Diseases or malignancies with an established or potential molecular link to dysregulation of EGFR activity are, for instance, described in *"*Mendelsohn and Baselga; Status of Epidermal Growth Factor Receptor Antagonists in the Biology and Treatment of Cancer, Journal of Clinical Oncology, 2787-2799"; "Mendelsohn and Baselga; Epidermal Growth Factor Receptor Targeting in Cancer, Seminars in Oncology, Vol 33, 369-385"; Irmer et al., EGFR kinase domain mutations - functional impact and relevance for lung cancer therapy, Oncogene, 1-9*;* Roche-Lima et al., EGFR targeting of solid tumors; Cancer Control, 2007, Vol 14 (3), 295-304) which all are, including the references cited therein, hereby incorporated into the present application by reference.

According to the present invention the treatment of the following EGFR dependent diseases, especially malignancies, with compounds of formula I, especially Compound A, is preferred:
- non small cell lung carcinoma
- head and neck cancer
- colorectal carcinoma
- breast cancer
- brain malignancies including glioblastoma
- prostate cancer
- bladder cancer
- renal cell carcinoma
- pancreas cancer
- cervical cancer
- esophageal cancer
- gastric cancer
- ovarian cancer
or any combination thereof.

The present invention relates to the use of acompound of formula I as described above, or a tautomer thereof, or a pharmaceutically acceptable salt, or a hydrate or solvate thereof for the manufacture of a pharmaceutical preparation for the treatment of an EGFR dependent disease.

Furthermore, the present invention relates to the use of a compound of formula I, especially 5-(2,6-di-morpholin-4-yl-pyrimidin-4-yl)-4-trifluoromethyl-pyridin-2-yamine for the manufacture of a pharmaceutical preparation for the treatment of an EGFR dependent disease or malignancy or a disease that has acquired resistance to agents that target EGFR family members.

The resistance to the treatment with an EGFR modulator can been acquired during treatment with said EGFR modulator or can be due to a mutation or mutations in the protein.

In particular, the present invention relates to the treatment of a disease or malignancy that is dependent on EGFR family members or has aquired resistance during treatment with an EGFR modulator, with compounds of formula I, especially Compound A or a pharmaceutically acceptable salt thereof. Possible EGFR modulators that upon treatment can result in resistance are, for instance, gefitinib, erlotinib, lapatinib, cetuximab, nimotuzumab, panitumumab, and trastuzumab.

A compound of the formula (I) may also be used for the treatment of EGFR dependent or EGFR acquired resistance diseases in combination with other active compounds for instance the combination partners as disclosed in WO07/084786, more preferred EGFR family targeting agents such us, and without limitation to gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922

The present invention also relates to a combination treatment of EGFR dependent diseases with a compounds of formula I, especially of a compound selected from the group consisting of (Compound A) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and/or ovarian cancer

In particular, the present invention relates to a combination of compound of formula I selected from the group consisting of 5-(2,6-di-morpholin-4-yl-pyrimidin-4-yl)-4-trifluoromethyl-pyridin-2-ylamine and an EGFR modulator selected from the group consisting of gefitinib, erlotinib, lapatinib, cetuximab, nimotuzumab, panitumumab, and trastuzumab, wherein the active ingredients are present in each case in free form or in the form of a pharmaceutically acceptable salt, and optionally at least one pharmaceutically acceptable carrier; for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

In another embodiment the present invention relates to a method of treating an EGFR dependent disease or a malignancy, preferably a malignancy, that has acquired resistance to EGFR kinase modulators during treatment with said EGFR modulator, comprising administering a therapeutically effective amount of a specific imidazoquinoline derivative of formula I, especially preferred 5-(2,6-di-morpholin-4-yl-pyrimidin-4-yl)-4-trifluoromethyl-pyridin-2-ylamine (Compound A) or a pharmaceutically acceptable salt thereof, alone or in combination with an EGFR modulator, to a warm-blooded animal in need thereof.

The diseases to be treated by this method are preferentially non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

In another embodiment the present invention relates to a pharmaceutical preparation for the treatment of an EGFR dependent disease or a disease that has acquired resistance during treatment with an EGFR modulator comprising a compound of formula I, especially preferred 5-(2,6-di-morpholin-4-yl-pyrimidin-4-yl)-4-trifluoromethyl-pyridin-2-ylamine (Compound A) or a pharmaceutically acceptable salt thereof and at least one pharmaceutically acceptable carrier, alone or in combination with an EGFR modulator.

The diseases to be treated by this pharmaceutical preparation are preferentially non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

In another embodiment the present invention relates to the use of a compound of formula I, especially preferred 5-(2,6-di-morpholin-4-yl-pyrimidin-4-yl)-4-trifluoromethyl-pyridin-2-y lamine (Compound A) or a pharmaceutically acceptable salt thereof for the treatment of an EGFR dependent disease or disease that has acquired resistance during treatment with an EGFR modulator.

The diseases to be treated by this compounds, alone or in combination with an EGFR modulator, are preferentially non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

A compound of the formula (I) may also be used to advantage in combination with known therapeutic processes, for example, hormone therapy or, especially, radiation. A compound of formula (I) may in particular be used as a radiosensitizer, especially for the treatment of tumors which exhibit poor sensitivity to radiotherapy.

Treatment in accordance with the invention may be symptomatic or prophylactic.

By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative, e.g. synergistic effect.

A compound of formula I can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The compounds of the invention may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising a compound of the invention in association with at least one pharmaceutical acceptable carrier or diluent may be manufactured in conventional manner by mixing with a pharmaceutically acceptable carrier or diluent.

The pharmaceutical compositions are comprising an amount effective in the treatment of one of the above-mentioned disorders, of a compound of formula I or an N-oxide or a tautomer thereof together with pharmaceutically acceptable carriers that are suitable for topical, enteral, for example oral or rectal, or parenteral administration and that may be inorganic or organic, solid or liquid. There are pharmaceutical compositions used for oral administration especially tablets or gelatin capsules that comprise the active ingredient together with diluents, for example lactose, dextrose, manitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilizers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectioning, dissolving or lyophilising lyophilizing processes, and comprise approximately from 1% to 99%, especially from approximately 1% to approximately 20%, active ingredient(s).

The present invention also provides the following embodiments as described in the following Paragraphs.

### Paragraphs:

1. Use of a compound of formula I, 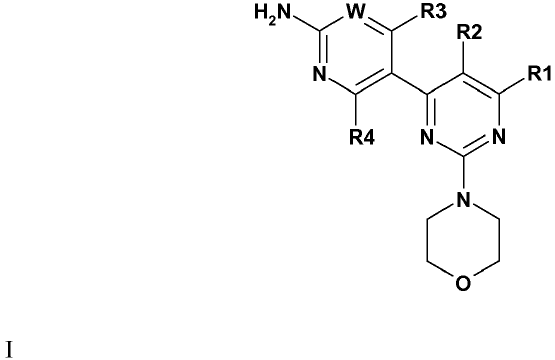 or a stereoisomer, tautomer, or pharmaceutically acceptable salt thereof, wherein, W is CR_{w} or N, wherein R_{w} is selected from the group consisting of
   (1) hydrogen,
   (2) cyano,
   (3) halogen,
   (4) methyl,
   (5) trifluoromethyl,
   (6) sulfonamido;
   R₁ is selected from the group consisting of
   (1) hydrogen,
   (2) cyano,
   (3) nitro,
   (4) halogen,
   (5) substituted and unsubstituted alkyl,
   (6) substituted and unsubstituted alkenyl,
   (7) substituted and unsubstituted alkynyl,
   (8) substituted and unsubstituted aryl,
   (9) substituted and unsubstituted heteroaryl,
   (10) substituted and unsubstituted heterocyclyl,
   (11) substituted and unsubstituted cycloalkyl,
   (12) -COR₁ₐ,
   (13) -CO₂R₁ₐ,
   (14) -CONR₁ₐR_{1b},
   (15) -NR₁ₐR_{1b},
   (16) -NR₁ₐCOR_{1b},
   (17) -NR₁ₐSO₂R_{1b},
   (18) -OCOR₁ₐ,
   (19) -OR₁ₐ,
   (20) -SR₁ₐ,
   (21) -SOR₁ₐ,
   (22) -SO₂R₁ₐ, and
   (23) -SO₂NR₁ₐR_{1b},
   wherein R₁ₐ, and R_{1b} are independently selected from the group consisting of
   (a) hydrogen,
   (b) substituted or unsubstituted alkyl,
   (c) substituted and unsubstituted aryl,
   (d) substituted and unsubstituted heteroaryl,
   (e) substituted and unsubstituted heterocyclyl, and
   (f) substituted and unsubstituted cycloalkyl;
   R₂ is selected from the group consisting
   (1) hydrogen,
   (2) cyano,
   (3) nitro,
   (4) halogen,
   (5) hydroxy,
   (6) amino,
   (7) substituted and unsubstituted alkyl,
   (8) -COR₂ₐ, and
   (9) -NR₂ₐCOR_{2b},
   wherein R₂ₐ, and R_{2b} are independently selected from the group consisting of
   (a) hydrogen, and
   (b) substituted or unsubstituted alkyl;
   R₃ is selected from the group consisting of
   (1) hydrogen,
   (2) cyano,
   (3) nitro,
   (4) halogen,
   (5) substituted and unsubstituted alkyl,
   (6) substituted and unsubstituted alkenyl,
   (7) substituted and unsubstituted alkynyl,
   (8) substituted and unsubstituted aryl,
   (9) substituted and unsubstituted heteroaryl,
   (10) substituted and unsubstituted heterocyclyl,
   (11) substituted and unsubstituted cycloalkyl,
   (12) -COR₃ₐ,
   (13) -NR₃ₐR_{3b},
   (14) -NR₃ₐCOR_{3b},
   (15) -NR₃ₐSO₂R_{3b},
   (16) -OR₃ₐ,
   (17) -SR₃ₐ,
   (18) -SOR₃ₐ,
   (19) -SO₂R₃ₐ, and
   (20) -SO₂NR₃ₐR_{3b},
   wherein R₃ₐ, and R_{3b} are independently selected from the group consisting of
   (a) hydrogen,
   (b) substituted or unsubstituted alkyl,
   (c) substituted and unsubstituted aryl,
   (d) substituted and unsubstituted heteroaryl,
   (e) substituted and unsubstituted heterocyclyl, and
   (f) substituted and unsubstituted cycloalkyl; and
   R₄ is selected from the group consisting of
   (1) hydrogen, and
   (2) halogen;
      for the manufacture of a pharmaceutical preparation for the treatment of an EGFR dependent disease.
2. The use according to Paragraph 1, where the compound of the formula I is 5-(2,6-di-morpholin-4-yl-pyrimidin-4-yl)-4-trifluoromethyl-pyridin-2-yamine (Compound A).
3. The use according to any one of Paragraphs 1 to 2, wherein the disease is a malignancy.
4. The use according to any one of Paragraphs 1 to 3, wherein said disease is resistant to the treatment with an EGFR modulator.
5. The use according to Paragraph 4, wherein resistance to the treatment with an EGFR modulator has been acquired during treatment with said EGFR modulator.
6. The use according to Paragraph 4, wherein the resistance is due to exon deletions and / or a mutation or mutations in the protein.
7. The use according to Paragraph 4 or 5, wherein the EGFR modulator is selected from the group consisting of gefitinib, erlotinib, lapatinib, cetuximab, nimotuzumab, panitumumab and trastuzumab.
8. The use according to any one of Paragraphs 1 to 3, together with an EGFR modulator.
9. The use according to Paragraph 8, wherein the EGFR modulator is selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.
10. The use according to any one of Paragraphs 1 to 9, wherein the disease to be treated is
   - non small cell lung carcinoma
   - head and neck cancer
   - colorectal carcinoma
   - breast cancer
   - brain malignancies including glioblastoma
   - prostate cancer
   - bladder cancer
   - renal cell carcinoma
   - pancreas cancer
   - cervical cancer
   - esophageal cancer
   - gastric cancer
   - ovarian cancer
      or any combination thereof.
11. Combination of a compound of formula I selected from the group consisting of 5-(2,6-di-morpholin-4-yl-pyrimidin-4-yl)-4-trifluoromethyl-pyridin-2-ylamine (Compound A) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and/or ovarian cancer.
12. A method of treating an EGFR dependent disease or a disease that has acquired resistance during treatment with an EGFR modulator comprising administering a therapeutically effective amount of a compound of formula I, according to any one of Paragraphs 1 to 2, to a warm-blooded animal in need thereof.
13. The method according to Paragraph 13, wherein the disease to be treated is a disease according to Paragraph 10.
14. The method according to Paragraph 12 or 13, wherein the compound of formula I is administered together with an EGFR modulator according to Paragraph 9.
15. A pharmaceutical preparation for the treatment of an EGFR dependent disease or a disease that has acquired resistance during treatment with an EGFR modulator comprising a compound of formula I, according to any one of Paragraphs 1 to 2 or a pharmaceutically acceptable salt thereof and at least one pharmaceutically acceptable carrier.
16. The pharmaceutical preparation according to Paragraph 15, wherein the disease to be treated is a disease according to Paragraph 10.
17. The pharmaceutical preparation according to Paragraph 15 or 16, comprising an EGFR modulator according to Paragraph 9.
18. Use of a compound of formula I, according to any one of Paragraphs 1 to 2 or a pharmaceutically acceptable salt thereof for the treatment of an EGFR dependent disease or disease that has acquired resistance during treatment with an EGFR modulator.
19. The use according to Paragraph 18, wherein the disease to be treated is a disease according to Paragraph 10.
20. The use according to Paragraph 18 or 19, together with an EGFR modulator according to Paragraph 9.

## Claims

1. The compound 5-(2,6-di-morpholin-4-yl-pyrimidin-4-yl)-4-trifluoromethyl-pyridin-2-ylamine or a pharmaceutically acceptable salt thereof for use in the treatment of an EGFR dependent disease, wherein the EGFR dependent disease is
• head and neck cancer
• breast cancer
• non small cell lung carcinoma
• colorectal carcinoma
• brain malignancies including glioblastoma
• prostate cancer
• bladder cancer
• renal cell carcinoma
• pancreas cancer
• cervical cancer
• esophageal cancer
• gastric cancer
• ovarian cancer
or any combination thereof.

2. The compound for use according to claim 1, wherein the disease is a malignancy.

3. The compound for use according to any one of claims 1 or 2, wherein said disease is resistant to the treatment with an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

4. The compound for use according to any one of claims 1 or 2, wherein said disease is resistant to the treatment with an EGFR modulator selected from the group consisting of gefitinib, erlotinib, lapatinib, cetuximab, nimotuzumab, panitumumab and trastuzumab.

5. The compound for use according to any one of claims 3 or 4, wherein resistance to the treatment with an EGFR modulator has been acquired during treatment with said EGFR modulator.

6. The compound for use according to claim 5, wherein the resistance is due to exon deletions and / or a mutation or mutations in the protein.

7. The compound for use according to any one of claims 1 to 3, together with an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

8. Combination of a compound 5-(2,6-di-morpholin-4-yl-pyrimidin-4-yl)-4-trifluoromethyl-pyridin-2-ylamine (Compound A) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of breast cancer, non small cell lung carcinoma, head and neck cancer, colorectal carcinoma, brain malignancies including glioblastoma, prostate cancer, bladder cancer, renal cell carcinoma, pancreas cancer, cervical cancer, esophageal cancer, gastric cancer and/or ovarian cancer.

9. A compound 5-(2,6-di-morpholin-4-yl-pyrimidin-4-yl)-4-trifluoromethyl-pyridin-2-ylamine according to claim 1 or a pharmaceutically acceptable salt thereof for use in the treatment of an EGFR dependent disease or a disease that has acquired resistance during treatment with an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the disease to be treated is breast cancer, non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer or ovarian cancer or any combination thereof.

10. The compound 5-(2,6-di-morpholin-4-yl-pyrimidin-4-yl)-4-trifluoromethyl-pyridin-2-ylamine according to claim 1 or a pharmaceutically acceptable salt thereof for use in the treatment according to claim 9, wherein said compound is administered together with an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

11. A pharmaceutical preparation for use in the treatment of an EGFR dependent disease or a disease that has acquired resistance during treatment with an EGFR modulator selected the group consisting of from gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922 and wherein the disease to be treated is breast cancer, non small cell lung carcinoma, head and neck cancer, colorectal carcinoma, brain malignancies including glioblastoma, prostate cancer, bladder cancer, renal cell carcinoma, pancreas cancer, cervical cancer, esophageal cancer, gastric cancer or ovarian cancer, or any combination thereof, comprising a compound 5-(2,6-di-morpholin-4-yl-pyrimidin-4-yl)-4-trifluoromethyl-pyridin-2-ylamine according to claim 1 or a pharmaceutically acceptable salt thereof and at least one pharmaceutically acceptable carrier.
